# EUROPEAN PATENT APPLICATION

(11) **EP 1 544 290 A1**
(43) Date of publication of application: **22.06.2005**
(21) Application number: 03258039.1
(22) Date of filing: 19.12.2003
(51) Int. Cl.: C12N 5/06

(54) **A cell system for generating somatic cells**

(71) Applicant: Fu, Yu-Show, Pei-Tou District, Taipei (TW); Wang, Hwai-Shi, Pei-Tou District, Taipei (TW)
(72) Inventor: Fu, Yu-Show, Pei-Tou District, Taipei (TW); Wang, Hwai-Shi, Pei-Tou District, Taipei (TW)
(74) Representative: Sarpi, Maurizio

(57) **Abstract**

The present invention relates to a cell system and a method for generating somatic cells.

## Description

### BACKGROUND OF THE INVENTION

### Field of the Invention

The present invention relates to a cell system and a method for generating somatic cells.

### Description of the Related Art

Many clinically common chronic neurodegenerative diseases, such as Parkinson's disease, Alzheimer's disease, Huntington's disease, and amyotrophic lateral sclerosis, are resulted from the degeneration and death of neurons in the central nervous system (CNS). Neurons, unlike epithelial and liver cells, cannot regenerate quickly. Although there are indeed stem cells present in the CNS, which can replicate and differentiate either under normal conditions or when neurons are damaged, their replication is quite slow and the proportion of neuronal differentiation is very low. Most of the stem cells in the CNS differentiate into glial cells and thus cannot rescue the neurodegenerative diseases.

Neuron transplantation has provided a direction for treating neurodegenerative diseases. Researchers have been trying to generate neurons from various stem cells both *in vitro* and *in vivo.* Stem cells are believed to possess certain characteristics, including self-renewal, pluripotency, proliferation, longevity and differentiation. Fetuses, umbilical cord blood and bone marrow are the most common sources of human stem cells for clinical applications. In fetuses, stem cells applicable in treating pathological changes of the central nervous system were mainly embryonic stem cells of blastocyst and neural stem cells (R.L. Rietze et al., 2001, *Nature* 412: 736-739). Researches have shown that after transplantation of embryonic stem cells (J.W. McDonald et al., 1999, *Nature Medicine* 5: 1410-1412) or neural stem cells (Q.L. Cao et al., 2001, *Experimental Neurology* 167: 48-58) for repairing a spinal trauma, these cells mainly differentiated into astrocytes and oligodendrocytes and only extremely few cells turned into neuronal cells. Apart from the difficulty in retrieving, the low survival rate after transplantation, and the extremely low rate for turning into neural cells, transplantation of fetus tissues is also hindered by disputes in the aspects of religion, law and ethic and therefore is not available to all.

The repair of damaged neural cells by direct implantation of haematopoietic stem cells of umbilical cord blood has yet to be studied. However, *in vitro* model showed that haematopoietic stem cells of umbilical cord blood cultured in 0.001% of β-Mercaptoethanol could be transformed into precursors of neural cells, and then 10% of these cells could be further induced to differentiate into neural cells and glial cells (J. R. Sanchez-Ramos *et al.,* 2001, *Experimental Neurology* 171: 109-115; and Y. Ha *et al.,* 2001, *Neuroreport* 12(16): 3523-3527). Since the conversion rate of umbilical cord blood stem cells into neuronal cells was relatively low, the research and application of umbilical cord blood mainly focused on haematological diseases.

Bone marrow contains hematopoietic stem cells that provide a continuous source for progenitors of red cells, platelets, monocytes, granulocytes, and lymphocytes. Moreover, bone marrow also contains cells that meet the criteria for stem cells of nonhematopoietic tissues. Hematopoietic stem cells from adult bone marrow have been reported to be able to differentiate into both microglia and macroglia in the brains of mice (M. A. Eglitis *et al.,* 1997, *Proc. Natl. Acad. Sci.* 94: 4080-4085). Nonhematopoietic stem cells from bone marrow stroma have been referred to as bone marrow stromal cells (BMSCs) or bone marrow mesenchymal cells. BMSCs are capable of differentiating into osteogenic, chondrogenic, adipogenic, myogenic and other lineages *in vitro* (M.F. Pittenger et al., 1999, Science 284: 143-147 and B. E. Petersen et al., 1999, Science 284: 1168- 1170). Recently, BMSCs (S. A. Azizi *et al.;* 1998, *Proc. Natl. Acad. Sci.* 95: 3908-3913; and G. C. Kopen *et al.,* 1999, *Proc. Natl. Acad. Sci.* 96: 10711-10716) have been reported to be able to differentiate into neurons *in vivo.*

All the above multipotent cells for generating neurons have one common problem: most of them differentiate into glial cells (astrocytes, oligodendrocytes *etc.)* instead of neurons. Only for *in vitro* culture of BMSCs, it has been observed that BME has a significant effect on neuronal differentiation, which induces about 80% of the cultured BMSCs to differentiate into neurons (D. Woodbury *et al.,* 2000, *Journal of Neuroscience Research* 61: 364-370). However, BME is toxic for proteins and thus unsuitable for use in human bodies (K. White et al., 1973, *Journal of Pharmaceutical Sciences* 62: 237-241). Therefore, there is still a need to explore novel sources of multipotent cells for generating neurons.

For many decades, millions of people have been suffering from heart attack that kills cardiac cells. The damaged area of the heart forms scar tissue, which impairs cardiac performance. Despite the development of improved therapies and the significant advances in the understanding of the basis of disease pathogenesis, number of death caused by cardiovascular diseases each year has not dramatically decreased to a satisfactory level. Therefore, the search for regenerated cardiac cells continues to occupy cardiovascular researchers hoping to find a way to replace the damaged heart tissue.

Since establishment of a multipotent cell may have significant impact on the study of early human cardiac differentiation, functional genomics, pharmacological testing, cell therapy, and tissue engineering, there is a growing demand for searching a new cell capable of differentiating into useful human cardiomyocytes without the least worry of the ethical, technical issues, such as those discussed above.

### SUMMARY OF THE INVENTION

In one aspect, the present invention provides a cell system for generating somatic cells. Specifically, the cell system of the invention comprises umbilical mesenchymal cells.

In another aspect, the present invention provides a method of generating somatic cells comprising the steps of culturing umbilical mesenchymal cells and inducing cell differentiation of the cultured umbilical mesenchymal cells.

In one embodiment, the cultured umbilical mesenchymal cells proliferate and differentiate into cells derived from the ectoderm.

In another embodiment, the cultured umbilical mesenchymal cells proliferate and differentiate into cells derived from the mesoderm.

In a further embodiment, the cultured umbilical mesenchymal cells proliferate and differentiate into cells derived from the endoderm.

### BRIEF DESCRIPTION OF THE DRAWINGS

Figure 1 shows (A) NF and (B) NeuN immunostaining of umbilical mesenchymal cells cultured for 48 hours in 2 mM β-mercaptoethanol (BME);
Figure 2 shows the morphology of umbilical mesenchymal cells cultured for 6 days in (A) 10%FBS-DMEM (Control) and (B) neuron-cultured medium;
Figure 3 shows (A) NF and (B) NeuN immunostaining of umbilical mesenchymal cells cultured in neuron-cultured medium for 6 days;
Figure 4 shows the ratios of umbilical mesenchymal cells expressing NF after various times of treatment with neuron-cultured medium;
Figure 5 shows the results of western blotting for GluR5, GluR6, GluR7 and KA2 of umbilical mesenchymal cells cultured in neuron-cultured medium;
Figure 6 shows immunostaining for (A) parvalbumin (PV) and (B) calbindin-D28K (CB) of umbilical mesenchymal cells cultured in neuron-cultured medium for 9 days;
Figure 7 shows (A) immunostaining and (B) western blotting for GAD of umbilical mesenchymal cells cultured in neuron-cultured medium for 6 days;
Figure 8(A) shows the altered cell density of umbilical mesenchymal cells induced by neuron-cultured medium on the 0, 3^{rd}, 6^{th} and 9^{th} day post-treatment (Scale bar: 100 µm);
Figure 8(B) shows quantitative cell density of umbilical mesenchymal cells treated with neuron-cultured medium using DAPI staining;
Figure 9 shows DAPI+BrdU double staining and NF+BrdU double staining for umbilical mesenchymal cells cultured in neuron-cultured medium for 3 days (3D) and 9 days (9D); and
Figure 10 shows photographic images illustrating expression of C-troponin I and F-actin filaments in the mesenchymal cells (A) not supplemented with a cell differentiating factor; and (B) after being supplemented with the cell differentiating factor.

### DETAILED DESCRIPTION OF THE EMBODIMENTS

It is surprisingly found that cultured umbilical mesenchymal cells can proliferate and differentiate into various somatic cells.

When used herein, the term "umbilical mesenchymal cells" refers to cells of the mesenchymal tissue in mammalian umbilical cord, preferably human umbilical cord.

According to the invention, umbilical mesenchymal cells are isolated from the umbilical cord and allowed to proliferate by culturing in a known cell culture medium. Culture media suitable for use in culturing umbilical mesenchymal cells according to the invention include, but are not limited to, Dulbecco's Modified Eagle Medium (DMEM). The cultured umbilical mesenchymal cells may be directly used to obtain somatic cells, or frozen in liquid nitrogen for later uses.

According to the invention, cultured umbilical mesenchymal cells are appropriately treated to trigger their differentiation. In one embodiment of the invention, the cultured umbilical mesenchymal cells differentiate into somatic cells from the ectoderm, including, but not limited to, neurons and retina. In another embodiment of the invention, the cultured umbilical mesenchymal cells differentiate into somatic cells from the mesoderm, including, but not limited to, muscle cells (skeletal muscle, smooth muscle, and cardiomyocytes and cardiovascular cells), osteogenic cells, chondrogenic cells, gastrointestinal organ and liver. In yet another embodiment of the invention, the cultured umbilical mesenchymal cells differentiate into somatic cells from the endoderm, including, but not limited to, mucous epithelium of gastrointestinal organ.

The neuron differentiation of cultured umbilical mesenchymal cells can be induced by adding suitable additives to the cultured cells. For examples, nerve growth factor (NGF) can be added to the culture of embryonic stem cells or neuronal stem cells to induce neuronal differentiation (AM Wobus et al., 1997, Journal of Molecular & Cellular Cardiology 29: 1525-1539.; E Cattaneo and R McKay, 1990, Nature 347: 762-765.). Other methods of inducing neuronal differentiation from bone marrow stromal cells include, but are not limited to, adding retinoic acid (RA), epidermal growth factor (EGF), and brain-derived neurotrophic factor (BDNF) (J Sanchez-Ramos et al., 2000, Experimental Neurology 164: 247-256). Another experiment has shown that β-mercaptoethanol (BME) induces differentiation of bone marrow stromal cells into neurons ( D Woodbury et al., 2000, Journal of Neuroscience Research 61: 364-370.)

The cardiomyocyte differentiation of cultured umbilical mesenchymal cells can be induced by adding suitable additives to the cultured cells. For examples, oxytocin can be added to the culture of embryonic stem cells to induce cardiomyocyte differentiation. (J Paquin et al., 2002, PNAS 99(14): 9550- 9555). Another method of inducing cardiomyocyte differentiation from bone marrow stromal cells is treatment with 5-azacytidine. Electron microscopy revealed a cardiomyocyte-like ultrastructure including typical sarcomeres and atrial granules (K Fukuda, 2001, Artificial Organs 25(3): 187- 193).

In one preferred embodiment of the present invention, umbilical mesenchymal cells cultured with a medium prepared by culturing neurons in a basic culture medium for 6-9 days differentiate into a high proportion (87%) of functional postmitotic neurons. The neuron-cultured medium is the subject matter of a co-pending patent application (Serial No. ).

In another preferred embodiment of the present invention, umbilical mesenchymal cells cultured in a medium supplemented with a cell differentiating factor such as 5-azacytidine differentiate into cardiomyocytes or cardiovascular cells.

The following assays are conducted to characterize the neurons produced by the method of the present invention.

### NF Immunocytochemistry

Neurofilament (NF) is the major cytoskeleton in neurons, which is responsible for adjusting the size, appearance and diameter of dendrites of neurons. Only neurons at the late developmental stage or mature neurons express NF. Immunostaining for NF is utilized to recognize neurons at the late stage.

### NeuN Immunocytochemistry

Neuron-specific nuclear protein (NeuN) is a specific protein existing in the nucleus of neurons in the central nervous system of vertebrates. NeuN binds to DNA *in vitro.* It is known that NeuN appears earlier than NF during the development of neurons. Therefore, immunostaining for NeuN is utilized to identify neurons at the early stage of transformation.

### Western Blotting for GluR5, GluR6, GluR7 and KA2

Glutamate is the major excitatory neurotransmitter in the mammalian CNS. Most neurons have glutamate receptors to receive excitatory signals. There are mainly two groups of glutamate receptors: the ionotropic type and metabotropic type. Ionotropic type receptors can be further divided by their different selective agonists into: NMDA (N-methyl-D-aspartate) receptors, AMPA (a-amino-3-hydroxy-5-methyl-4-isoxazole propionic acid) receptors, and KA (kainate) receptors. KA receptors are formed by subunits GluR5, GluR6, GluR7, KA1 and KA2. Therefore, western blotting for GluR5, GluR6, GluR7 and KA2 is utilized to confirm the ability of neurons to synthesize functional proteins.

### Ca²⁺-binding Protein Immunocytochemistry

Ca²⁺ plays an important role in the signal transduction pathway of cells. However, most Ca²⁺s do not act alone in the cell, but are bound by Ca²⁺-binding proteins. Ca²⁺-binding proteins can buffer the concentration of Ca²⁺ in the cell and further regulate the activities of Ca²⁺. There are many Ca²⁺-binding proteins present in the CNS, and those with the widest distribution are parvalbumin (PV), calbindin-D28K (CB) and calretinin. Both PV and CB belong to the EF-hand type Ca²⁺-binding protein family. During the development of the nervous system of embryo, PV and CB are considered as the Ca²⁺-binding proteins specifically expressed by neurons. Therefore, immunostaining for PV and CB is utilized to confirm the ability of neurons to synthesize functional proteins.

### BrdU and DAPI Double Labeling

DAPI (4',6-diamidino-2-phenylindole dihydrochloride) is a fluorescent dye able to cross freely through the cell and nuclear membranes and bind to the DNAs in the nucleus, and is usually used to quantify the number of cells. Moreover, during replication, cells need to synthesize an additional set of chromosomes, and will incorporate the added BrdU (bromodeoxyuridine), which is an analog of thymidine, into the synthesized chromosomes. Thus the observation of BrdU incorporation is utilized to confirm the replication of cells. BrdU and DAPI double labeling can confirm the cell ratio of the replication.

### BrdU and NF Double Labeling

As described above, Immunostaining for NF is utilized to confirm the identity of neurons, and BrdU labeling is utilized to confirm the replication of cells. NF and BrdU double labeling can identify the replication ability of neurons.

### GAD Immunocytochemistry

The synthesis of GABA, one of the neurotransmitters, from glutamic acid is catalyzed by the enzyme glutamic acid decarboxylase (GAD). Therefore, immunostaining for GAD is utilized to confirm the ability of neurons to synthesize the neurotransmitter GABA.

The neurons generated from the method of the present invention can be used in the treatment of neurodegenerative diseases, such as Parkinson's disease, Alzheimer's disease, Huntington's disease, and amyotrophic lateral sclerosis or neuronal death induced from stroke and trauma.

The following assays are conducted to characterize the cardiomyocytes and cardiovascular cells produced by the method of the present invention.

### Cardiac Troponin I/F-actin Immunocytochemistry

Using immunocytochemistry, the presence of cardiac specific proteins and their spatial organization can be studied in the mesenchymal cells forming contracting cardiomyocytes. Specific cardiac cell markers, such as cardiac troponin I, have been shown (Kehat et al., 2001.) to be positively stained in the contracting cardiomyocytes, confirming the cardiomyocytic, rather than skeletal, nature of the cells. Furthermore, to show that the mesnchymal cells are organized in an orderly aggregate that resembles the cellular organization in the cardiac tissue, the cells were also stained for F-actin, which is an essential cell cytoskeletal protein that maintains the intact cell structure.

The present invention is further illustrated by the following examples, which should not be taken as limitations to the scope of the present invention.

### Example 1 Preparation of Human Umbilical Mesenchymal Cells

Human umbilical cords were obtained from a clinic with child delivery service, Tsai's Obstetrical Clinic (72, Sec. 1, Shi Pai Rd., Beitou, Taipei). The umbilical cords were collected by aseptic manipulation and stored in HBSS (Biochrom L201-10) under 4°C for no more than 24 hours.

The umbilical cord was first soaked in 75% ethanol for 30 seconds for sanitization. The sanitized umbilical cord was placed in Ca²⁺/Mg²⁺ free buffer (CMF, Gibco 14185-052) in a germ-free laminar flow, cut lengthwise with an autoclaved tool, and from which the blood vessels and mesenchymal tissue (Wharton's jelly) were removed. The mesenchymal tissue was diced into cubes of about 0.5 cm³ and centrifuged at 250 xg for 5 minutes. The supernatant was removed, and the precipitate (mesenchymal tissue) was washed twice by adding a proper amount of serum-free DMEM (Gibco 12100-046) and centrifuging at 250x g for 5 minutes. The mesenchymal tissue was treated with Collagase at 37°C for 14 to 18 hours, washed, and then treated with 2.5% trypsin (Gibco 15090-046) under agitation at 37°C for 30 minutes. FBS (Hyclone SH30071.03) was added to the mesenchymal tissue to quench the activity of trypsin. At this time, the mesenchymal tissue has become mesenchymal cells. The mesenchymal cells were treated with 10% FBS-DMEM to be dispersed and counted. The mesenchymal cells can now be directly used for culturing.

### Example 2 Transformation of Umbilical Mesenchymal Cells into Neurons with β-mercaptoethanol (BME)

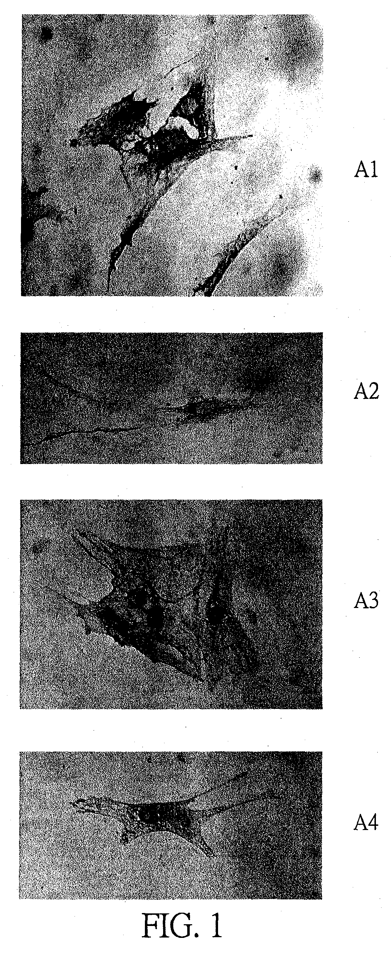

Umbilical mesenchymal cells obtained in Example 1 were cultured in 10%FBS-DMEM for 72 hours. Two mM BME was added to the culture and the cells continued to be cultured for 72 hours. The results are shown in Fig. 1.

As can be seen from Fig. 1 (A1 and A2), umbilical mesenchymal cells cultured in 2 mM BME for 72 hours expressed NF, suggesting that they have been transformed into neurons. As shown in Fig. 1 (Bl and B2), umbilical mesenchymal cells cultured in 2 mM BME for 72 hours expressed NeuN, suggesting that they have been transformed into neurons.

### Example 3 Transformation of Umbilical Mesenchymal Cells into Neurons with Neuron-Cultured 10%FBS-DMEM

### (a) Preparation of Neuron-Cultured 10%FBS-DMEM

The neurons used for preparing the neuron-cultured 10%FBS-DMEM were obtained from the brain of a Sprague-Dawley rat aged 7 days (the Animal Center of National Yang-Ming University, Taipei, Taiwan, ROC).

A 50-ml centrifugation tube containing 24 ml 10% FBS-DMEM was placed in a 37°C incubator before the subsequent operation.

The rat was given 0.3 ml 10% chloride hydrate by *i.p.* injection. After 3 to 4 minutes, the whole body of the totally anesthetized rat was sanitized with 75% ethanol. In a germ-free laminar flow, the brain of the rat was removed and placed in CMF. The brain was centrifuged at 900 rpm for 5 minutes. The supernatant was removed, and 10% FBS-DMEM was added to the precipitate (brain tissue). The brain tissue was pipetted 15 times to be dispersed into single cells. The cell dispersion was added to the 50-ml centrifugation tube prepared above, and mixed thoroughly with the 10%FBS-DMEM inside. The mixture was added into the poly-L-lysine coated wells of a 24-well plate and cultured in the 37°C incubator. The next day, Arac was added to the culture to a final concentration of 2 µM.

On the fifth day of culturing, the culture medium was removed to be used for culturing umbilical mesenchymal cells.

### (b) Transformation of Umbilical Mesenchymal Cells into Neurons

Umbilical mesenchymal cells obtained in Example 1 were cultured in the neuron-cultured 10%FBS-DMEM prepared in the above step (a) or in 10%FBS-DMEM (Control) in a 37°C incubator. Cultured cells were collected on the 3rd, 6th, 9th and 12th days.

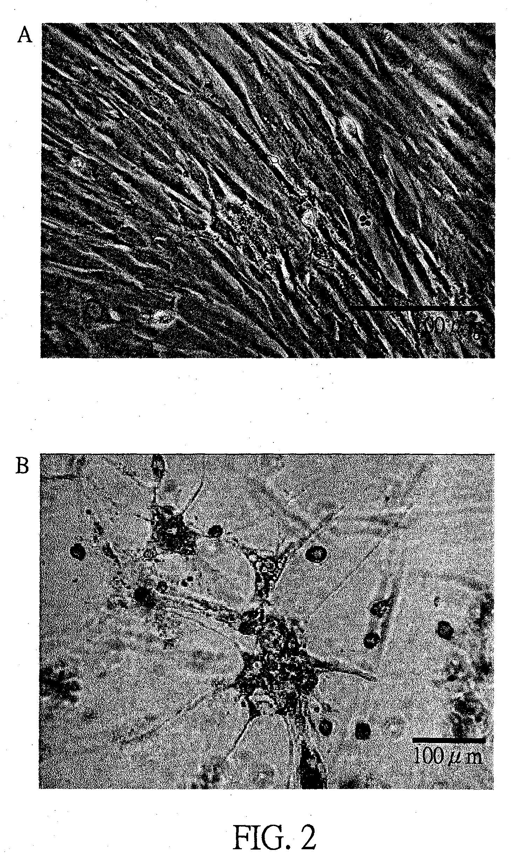

As shown in Fig. 2(A), the morphology of umbilical mesenchymal cells culture in 10%FBS-DMEM for 6 days (control) presented a spindle morphology and were closely attached to each other due to proliferation. Umbilical mesenchymal cells cultured in the neuron-cultured 10%FBS-DMEM for 6 days exhibited neuronal phenotype, including cell body retraction, process elaboration, and cell cluster (Fig. 2B).

### Example 4 Assays for the Characterization of Neuronal Differentiation

The cells collected in Example 3 were subjected to the following assays and their results are described below:

### (I) NF Immunostaining:

Cells growing on the cover glass for 6 days were washed with 0.1 M phosphate buffer (0.1 M PB, pH 7.4) for 5 minutes twice; fixed with the fixing solution (4% paraformaldehyde in 0.1 M PB) for 20 minutes; washed with 0.1 M PB for 5 minutes three times; and then treated with the blocking solution (0.05% Triton X-100, 5% normal goat serum and 3% bovine serum albumin) for 30 minutes to prevent non-specific antibody-antigen binding.

The treated cells were then reacted with primary antibodies (rabbit anti-neurofilament 200 polyclonal IgG, 1:250 diluted, Chemicon AB1982) at 4°C for at least 12 to 18 hours; washed with 0.1 M PBS for 5 minutes three times; reacted with secondary antibodies (Biotin-conjugated goat anti-rabbit-IgG, 1:1000 diluted, Sigma b-8895) at room temperature for 1 hour; washed again with 0.1 M PBS for 5 minutes three times; reacted with avidin-biotinylated-horseradish peroxidase complex (ABC KIT, Vectorlabs PK-4000) at room temperature for 1 hour; washed with 0.1 M PBS for 5 minutes three times; and then developed with 3,3'-diaminobenzidine (5 mg DAB, 3.5 µl 30% H₂O₂ in 10 ml 50 mM Tris Buffer).

The obtained sample was dehydrated with ethanol of progressively increasing concentrations of 50%, 70%, 80%, 90%, 95% and 100%, as well as xylene, each for 5 minutes. The cover glass was sealed onto a slide with permount, and observed under an optical microscope (Olympus BH-2).

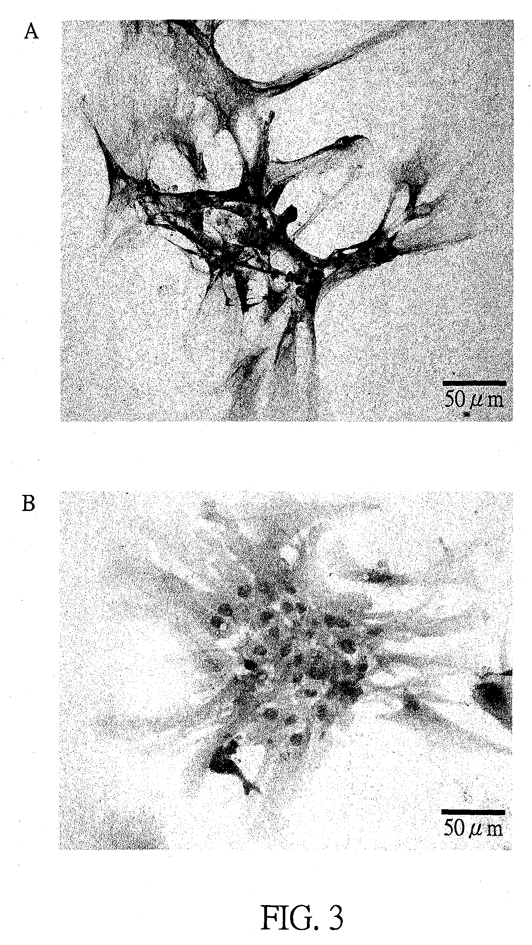

As shown in Fig. 3(A), umbilical mesenchymal cells cultured in the neuron-cultured 10%FBS-DMEM for 6 days expressed NF, suggesting that they have been transformed into neurons.

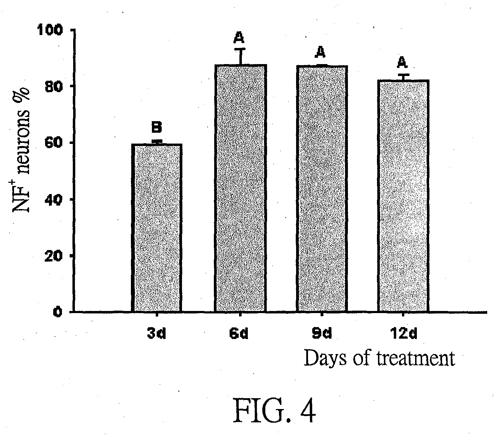

The ratio of umbilical mesenchymal cells expressing NF after various times of treatment with neuron-cultured 10%FBS-DMEM was determined. As shown in Fig. 4, 59.4% of the umbilical mesenchymal cells expressed NF on the 3rd day post treatment, and the ratio of cells expressing NF reached maximum, 87.4%, on the 6th day post treatment. This ratio neither increased nor decreased along with the elongation of time (n=3, one way ANOVA followed by LSD test; P<0.01).

### (II) NeuN Immunostaining:

Cells growing on the cover glass for 6 days were washed with 0.1 M phosphate buffer (0.1 M PB, pH 7.4) for 5 minutes twice; fixed with the fixing solution (4% paraformaldehyde in 0.1 M PBS) for 20 minutes; washed with 0.1 M PBS for 5 minutes three times; and then treated with the blocking solution (0.05% Triton X-100, 5% normal goat serum and 3% bovine serum albumin) for 30 minutes to prevent non-specific antibody-antigen binding.

The treated cells were then reacted with primary antibodies (mouse anti-neuronal nuclei monoclonal IgG, 1:100 diluted, Chemicon MAB377) at 4°C for at least 36 to 42 hours; washed with 0.1 M PBS for 5 minutes three times; reacted with secondary antibodies (Biotin-conjugated goat anti-mouse-IgG, 1:250 diluted, Chemicon AP124B) at room temperature for 1 hour; washed again with 0.1 M PBS for 5 minutes three times; reacted with avidin-biotinylated-horseradish peroxidase complex (ABC KIT, Vectorlabs PK-4000) at room temperature for 1 hour; washed with 0.1 M PBS for 5 minutes three times; and developed with 3,3'-diaminobenzidine (5 mg DAB, 3.5 µl 30% H₂O₂ in 10 ml 50 mM Tris Buffer).

The obtained sample was dehydrated with ethanol of progressively increasing concentrations of 50%, 70%, 80%, 90%, 95% and 100%, as well as xylene, each for 5 minutes. The cover glass was sealed onto a slide with permount, and observed under an optical microscope (Olympus BH-2).

As shown in Fig. 3(B), umbilical mesenchymal cells cultured in the neuron-cultured 10%FBS-DMEM for 6 days expressed NeuN, suggesting that they have been transformed into neurons.

### (III) Western Blotting for GluR5, GluR6, GluR7 and KA2:

### (a) Preparation of Cell Membrane

Cell Membrane was prepared from umbilical mesenchymal cells treated with neuron-cultured 10%FBS-DMEM for different times and the brain of a 7-day aged rat. Cultured cells were washed with 0.1 M phosphate buffer for 3 minutes twice. Tris-HCl buffer (50mM, pH 7.4) was added to the cells at 4°C , and the cells were scraped off from the culture plate with a scraper. The scraped cells were put in a 15 ml Teflon-glass homogenizer, grinded twenty times to be homogenized, and then centrifuged at 30,000 g for 30 minutes at 4C. The obtained pellet, which was mostly cell membrane, was grinded again and dissolved in a suitable volume of Tris-HCl buffer, and stored in a -20°C refrigerator for later use.

### (b) Determination of Protein Concentration

In order to be used in the western blotting assay, the concentration of proteins in each sample obtained above was determined by measuring the absorption at 595 nm. The measurement of protein concentration was conducted based on the Bradford method (MM. Bradford, 1976, Analytical Biochemistry 72: 248-254.).

### (c) Electrophoresis

The proteins in the samples were separated by 20% SDS-PAGE. The separated proteins in the gel were then transferred onto a PVDF paper (NEW, NEF1002) for conducting the western blotting assay.

### (d) Western Blotting

The PVDF paper containing the proteins was washed with TBS solution (0.9% NaCl in 50 mM Tris-HCl, pH 7.4) for 30 minutes twice. Blocking was conducted with 5% skimmed milk powder dissolved in TBS buffer for 60 minutes. Then the PVDF paper was immersed in the blocking solution (0.05% Triton-X 100, 5% normal goat serum and 3% BSA) for 60 minutes, washed with TBS buffer, and reacted with primary antibodies (rabbit anti-KA2 polyclonal IgG, 1:1800 diluted, UPSTATE 06-315; goat anti-GluR5 polyclonal IgG, 1:30 diluted, Santa Cruz sc-7617; goat anti-GluR6 polyclonal IgG, 1:30 diluted, Santa Cruz sc-7618; goat anti-GluR7 polyclonal IgG, 1:30 diluted, Santa Cruz sc-7620; and rabbit anti-glutamate decarboxylase polyclonal IgG, 1:1500 diluted, Chemicon AB108) at 4°C for 12 to 18 hours.

After the reaction was completed, the PVDF paper was washed with TTBS (0.05% Tween-20 in TBS) for 30 minutes twice, immersed in the blocking solution for 60 minutes, and then reacted with secondary antibodies (Biotin-anti-goat/sheep IgG for GluR5/6/7, 1:200 diluted, Sigma B-3148; and Biotin-anti-rabbit IgG for KAR2/GAD, 1:250 diluted, Chemicon AP187B) at room temperature for 1 hour. The reacted PVDF paper was washed with TTBS for 30 minutes twice, reacted with avidin-biotinylated-horseradish peroxidase complex (ABC KIT, Vectorlabs PK-4000) at room temperature for 1 hour, washed again with TTBS for 30 minutes twice, and finally developed with DAB (5 mg DAB, 3.5 µl 30% H₂O₂ in 10 ml Tris-HCl, pH7.4).

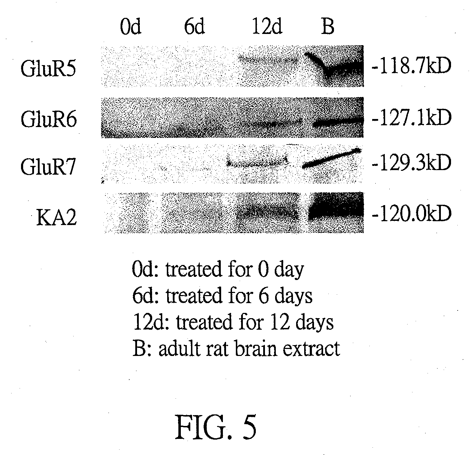

As shown in Fig. 5, kainate receptor subunits were not expressed in umbilical mesenchymal cells before the treatment of neuron-cultured 10%FBS-DMEM. On the 6^{th} day of treatment with neuron-cultured 10%FBS-DMEM, kainate receptor subunits, including GluR6, GluR7 and KA2, were expressed at a small quantity and GluR5, GluR6, GluR7 and KA2 were expressed significantly on the 12^{th} day.

### (IV) Parvalbumin (PV) and Calbindin-D28K (CB) Immunostaining:

Cells growing on the cover glass for 6 days were washed with 0.1 M phosphate buffer (0.1 M PBS, pH 7.4) for 5 minutes twice; fixed with the fixing solution (2% paraformaldehyde and 7.5% piric acid in 0.1 M PBS) for 20 minutes; washed with 0.1 M PBS for 5 minutes three times; and treated with the blocking solution (0.05% Triton X-100, 5% normal goat serum and 3% bovine serum albumin) for 30 minutes to prevent non-specific antibody-antigen binding.

The treated cells were then reacted with primary antibodies (goat anti-parvalbumin polyclonal IgG, 1:40 diluted, Santa Cruz sc-7449; and goat anti-calbindin polyclonal IgG, 1:40 diluted, Santa Cruz sc-7691) at 4°C for at least 12 to 18 hours; washed with 0.1 M PBS for 5 minutes three times; reacted with secondary antibodies (Biotin-conjugated mouse anti-goat/sheep monoclonal IgG, 1:250 diluted, Sigma B3148) at room temperature for 1 hour; washed again with 0.1 M PBS for 5 minutes three times; reacted with avidin-biotinylated-horseradish peroxidase complex (ABC KIT, Vectorlabs PK-4000) at room temperature for 1 hour; washed with 0.1 M PBS for 5 minutes three times; and developed with 3,3'-diaminobenzidine (5 mg DAB, 3.5 µl 30% H₂O₂ in 10 ml 50 mM Tris Buffer).

The obtained sample was dehydrated with ethanol of progressively increasing concentrations of 50%, 70%, 80%, 90%, 95% and 100%, as well as xylene, each for 5 minutes. The cover glass was sealed onto a slide with permount, and' observed under an optical microscope (Olympus BH-2).

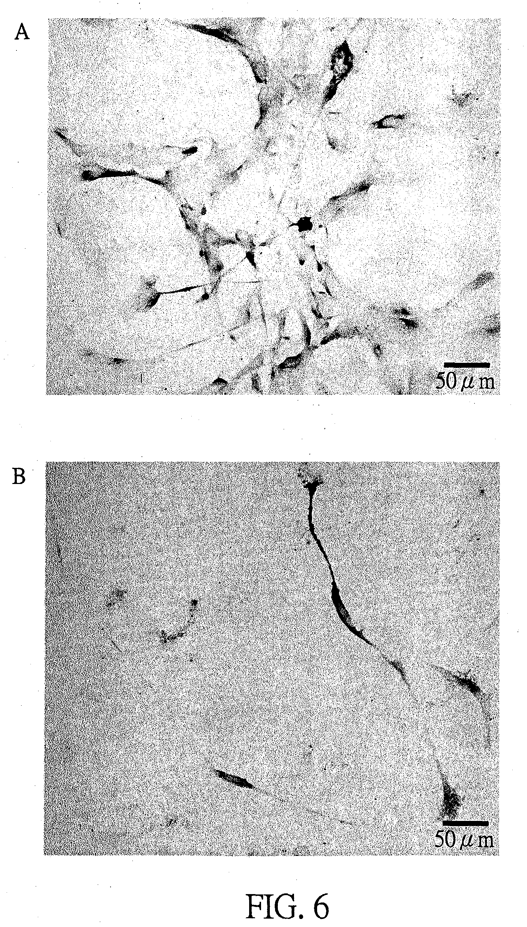

As shown in Fig. 6, umbilical mesenchymal cells cultured in the neuron-cultured 10%FBS-DMEM for 9 days expressed both parvalbumin (Fig. 6A) and calbindin-D28K (Fig. 6B), suggesting that they have been transformed into neurons capable of synthesizing the two Ca²⁺- binding proteins.

### (V) GAD Immunostaining and Western Blotting:

Cells growing on the cover glass for 6 days were washed with 0.1 M phosphate buffer (0.1 M PB, pH 7.4) for 5 minutes twice; fixed with the fixing solution (2% paraformaldehyde and 7.5% piric acid in 0.1 M PBS) for 20 minutes; washed with 0.1 M PBS for 5 minutes three times; and treated with the blocking solution (0.05% Triton X-100, 5% normal goat serum and 3% bovine serum albumin) for 30 minutes to prevent non-specific antibody-antigen binding.

The treated cells were then reacted with primary antibodies (rabbit anti-glutamate decarboxylase polyclonal IgG, 1:1500 diluted, Chemicon AB108) at 4°C for at least 12 to 18 hours; washed with 0.1 M PBS for 5 minutes three times; reacted with secondary antibodies (Biotin-conjugated goat anti-rabbit-IgG, 1:300 diluted, Sigma b-8895) at room temperature for 1 hour; washed again with 0.1 M PBS for 5 minutes three times; reacted with avidin-biotinylated-horseradish peroxidase complex (ABC KIT, Vectorlabs PK-4000) at room temperature for 1 hour; washed with 0.1 M PBS for 5 minutes three times; and developed with 3,3'-diaminobenzidine (5 mg DAB, 3.5 µl 30% H₂O₂ in 10 ml 50 mM Tris Buffer).

The obtained sample was dehydrated with ethanol of progressively increasing concentrations of 50%, 70%, 80%, 90%, 95% and 100%, as well as xylene, each for 5 minutes. The cover glass was sealed onto a slide with permount, and observed under an optical microscope (Olympus BH-2).

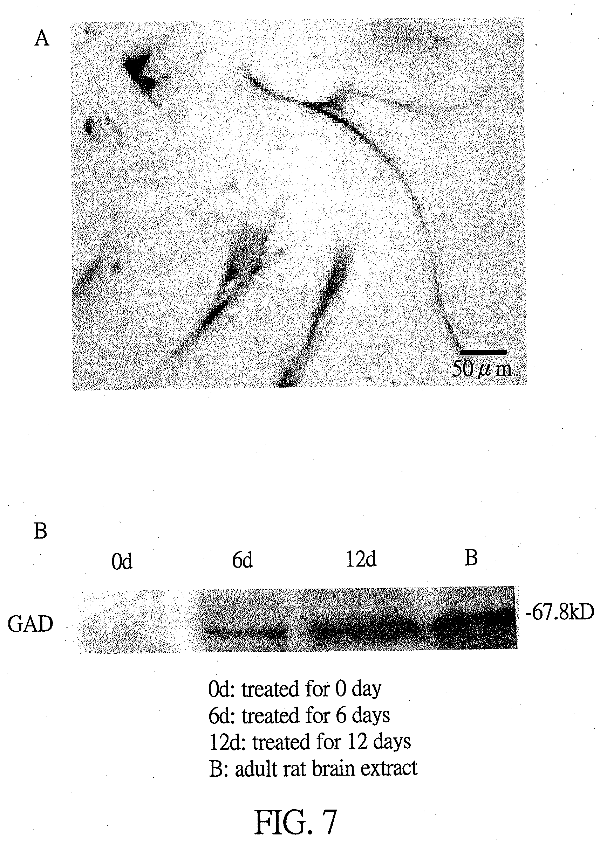

As shown in Fig. 7(A), umbilical mesenchymal cells cultured in the neuron-cultured 10%FBS-DMEM for 6 days expressed GAD, suggesting that they have been transformed into neurons capable of synthesizing the neurotransmitter GABA. As shown in Fig. 7(B), the capability of umbilical mesenchymal cells to express GAD was induced on the 6^{th} day post-treatment and the GAD expression level was significantly increased on the 12^{th} day.

The results in (III), (IV) and (V) prove that the neurons derived by the method of the present invention are functional and capable of synthesizing neuron-characteristic proteins and neurotransmitters.

### (VI) DAPI staining:

After treatment, the cells were washed twice with 0.1M phosphate buffer for 5 minutes each and then stained with 50 µg/ml DAPI for 30 minutes. The cells were then washed thoroughly with Tris buffer (Tris-HCl 50 mM pH=7.3) and mounted with mounting medium (Tris:glycerol = 1:1) to observe and count cells under fluorescence microscope.

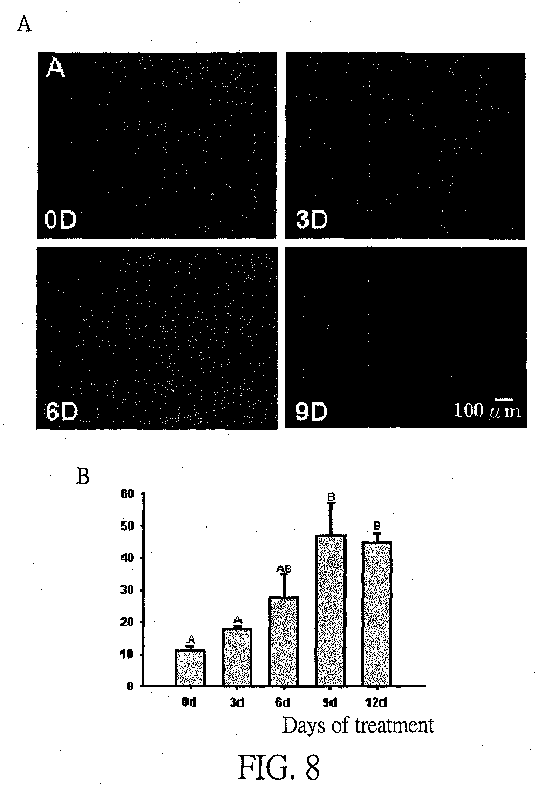

In Fig. 8(A), photomicrographs showing the cell density, DAPI (blue) was performed to determine the number at the 0, 3^{rd}, 6^{th} and 9^{th} day post-treatment. Fig. 8(B) shows the cell density of umbilical mesenchymal cells after various periods of treatment in neuron-cultured 10%FBS-DMEM. At various post treatment time points, DAPI staining was performed to determine the alternation in the cell density. The results indicated that the cell density on the 9th day post-treatment was significantly higher than that on the 3rd day (n=3, one way ANOVA followed by LSD test; P<0.01).

### (VII) BrdU and DAPI Double Staining:

Cells growing on the cover glass were given BrdU (Sigma B-5002, prepared as a 50 mM stock solution) of a final concentration of 50 µM and allowed to grow for 24 more hours. The cells were washed with 0.1 M phosphate buffer (0.1µM PBS, pH 7.4) for 5 minutes twice; fixed with the fixing solution (70% ethanol in 50 mM glycine buffer, pH 2.0) at -20°C for 30 minutes; washed with 0.1 M PBS for 5 minutes three times; and treated with the blocking solution (0.05% Triton X-100, 5% normal goat serum and 3% bovine serum albumin) for 30 minutes to prevent non-specific antibody-antigen binding.

The treated cells were then reacted with primary antibodies (mouse anti-BrdU monoclonal IgG, Chemicon MAB3222, 1:100 diluted with 0.66 mM CaCl₂ and 1 mM β-mercaptoethanol in 66 mM Tris buffer) at 4°C for at least 36 to 42 hours; washed with 0.1 M PBS for 5 minutes three times; reacted with secondary antibodies (Fluorescein-conjugated goat anti-mouse-IgG, 1:50 diluted, Chemicon AP124F) and 1 mg/ml DAPI (Sigma D9542) at room temperature for 1 hour; and washed again with 0.1 M PBS for 5 minutes three times. The cover glass was attached onto a slide with mounting medium (Vector H-1000), sealed with nail-enamel and observed under a fluorescence microscope (Olympus BX50). The total cell number and BrdU-labeled cell number within unit area were counted.

### (VIII) BrdU and NF Double Staining:

Cells growing on the cover glass were given BrdU (Sigma B-5002, prepared as a 50 mM stock solution) of a final concentration of 50 µM and allowed to grow for 24 more hours. The cells washed with 0.1 M phosphate buffer (0.1 M PBS, pH 7.4) for 5 minutes twice; fixed with the fixing solution (70% ethanol in 50 mM glycine buffer, pH 2.0) at -20°C for 30 minutes; washed with 0.1 M PBS for 5 minutes three times; and treated with the blocking solution (0.05% Triton X-100, 5% normal goat serum and 3% bovine serum albumin) for 30 minutes to prevent non-specific antibody-antigen binding.

The treated cells were then reacted with a primary antibody (mouse anti-BrdU monoclonal IgG, Chemicon MAB3222, 1:100 diluted with 0.66 mM CaCl₂ and 1 mM β-mercaptoethanol in 66 mM Tris buffer) at 4°C for at least 12 to 18 hours; reacted with another primary antibody (rabbit anti-neurofilament polyclonal IgG, 1:250 diluted, Chemicon AB1982) at 4°C for at least 12 to 18 hours; washed with 0.1 M PBS for 5 minutes three times; reacted with secondary antibodies (Fluorescein-conjugated goat anti-mouse-IgG, 1:50 diluted, Chemicon AP124F; and Rhodamine-conjugated goat anti-rabbit-IgG, 1:50 diluted, Chemicon AP132R) at room temperature for 1 hour; and washed again with 0.1 M PBS for 5 minutes three times. The cover glass was attached onto a slide with mounting medium (Vector H-1000), sealed with nail-enamel and observed under a fluorescence microscope (Olympus BX50).

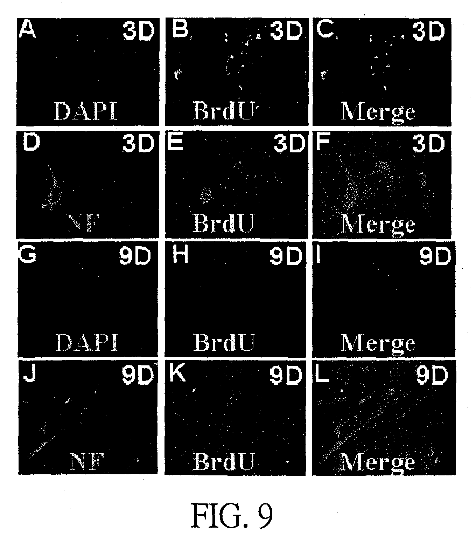

Fig 9 shows the results of the proliferation assay of umbilical mesenchymal cells treated with neuron-cultured 10%FBS-DMEM for 3 days (A-F). The majority of the cells labeled with DAPI (blue in A) were BrdU-positive (green in B). (C) is the merged result of (C) and (D). On the 3^{rd} day post treatment, these cells, although have already differentiated into cells expressing NF (red in D), were still labeled with BrdU (green in E). (F) is the merged result of (D) and (E). The cells on the 9^{th} day post treatment with neuron-cultured 10%FBS-DMEM lost their proliferation capability (G-L). All the cells labeled with DAPI (blue in G) were BrdU-negative (green in H). (I) is the merged result of (G) and (H). On the 9^{th} day post-treatment, these cells, which have differentiated into cells expressing NF (red in J), were almost not labeled with BrdU (green in K). (L) is the merged result of (J) and (K). In summary, most of the cells have been transformed into neurons and stopped proliferating on the 9th day, suggesting that the neurons on the 9th day have entered the postmitotic stage.

The results in (VII) and (VIII) demonstrate that the umbilical mesenchymal cells cultured with the neuron-cultured 10%FBS-DMEM can proliferate and differentiate into neurons.

### Example 5 Transformation of Umbilical Mesenchymal Cells into Cardiomyocytes and Cardiovascular cells

Umbilical mesenchymal cells obtained in Example 1 were cultured in a cell growing medium of DMEM supplemented with about 4.5 g/L of glucose and about 10%FBS. After the cells have attached to the bottom of the cell culture plate, the cell growing medium was then replaced by a cell differentiating medium, which was prepared by supplementing the cell growing medium with 3 µM 5-azacytidine as a cell differentiating factor. Alternatively, the cell differentiating factor may be directly added to the culture.

The umbilical mesenchymal cells were cultured in the cell differentiating medium for 24 hours. The cell differentiating medium was then replaced by the cell growing medium to prevent intoxication of the cells caused by the long exposure to 5-azacytidine. The differentiated cells were further maintained in the cell growing medium for about 1-3 weeks to ensure the viability and optimal differentiation of the cells. The culture medium was replaced with fresh medium every three days.

### Example 6 Assays for the Characterization of Cardiac Differentiation

Cells cultured on cover slips following the procedures in Example 5 were fixed with a fixing agent (such as paraformaldehyde) to preserve the cells in a the viable state. The cells fixed on the cover slips were washed with an isotonic saline solution before cell differentiation was blocked and the cells were permeabilized by using a blocking agent containing a cell-permeabilizing agent (such as 0.1% Triton X-100) for 15 minutes at room temperature. Then, monoclonal antibodies specifically recognizing and binding cardiac-specific the C-troponin I epitope and F-actin filaments (Troponin I-C Affinity-purified Goat Polyclonal Antibody, supplied by SantaCruz Biotechnology; and F-actin was stained by FITC-conjugated Phalloidin, supplied by Molecular Probes ) (the primary antibodies) were added at a 1:100 dilution to the differentiated cells fixed on the cover slips. After incubation for 1 hour, any non-specific binding by the primary antibodies was removed by washing with the saline solution.

Next, secondary antibodies conjugated to fluorescence or any visible dyes readily detectable by an optical instrument such as a confocal laser scanning microscope, a fluorescence microscope, or an optical microscope, Rhodamine (TRITC)-conjugated AffiniPure Donkey Anti-Goat IgG (supplied by Jackson ImmunoResearch Lab) were added at a 1:100 dilution to bind the host species of the primary antibodies. After incubation for 1 hour, any non-specific binding by the secondary antibodies is removed by washing with the saline solution. Then, the labeled cells were mounted on the cover slide using a mounting medium and viewed with a confocal laser scanning microscope.

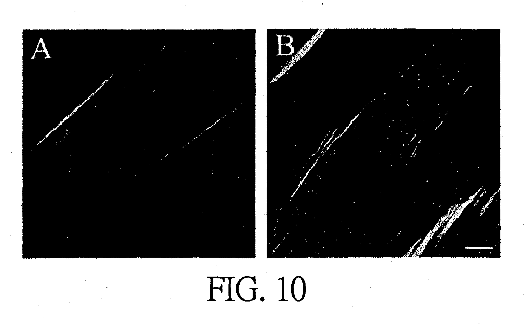

As shown in Fig. 10(A) and Fig. 10(B), the differentiated cells exhibited significant expression of C-troponin I compared to the cells not treated with the cell differentiating factor, indicating that the umbilical mesenchymal cells have successfully differentiated into human cardiomyocytes or cardiovascular cells. And F-actin expression in the differentiated cells further confirmed that the differentiated cells exhibited a specific cell attachment pattern similar to that of the cardiomyocytes or cardiovascular cells.

From the above descriptions, persons skilled in the art can easily acknowledge the essential characteristics of the present invention and, without departing from the spirit and scope thereof, can make various changes and modifications of the invention to adapt it to various usages and conditions.

## Claims

1. A cell system for generating somatic cells comprising umbilical mesenchymal cells isolated from mesenchymal tissue in an umbilical cord.

2. The cell system of Claim 1, wherein the umbilical mesenchymal cells are mammalian umbilical mesenchymal cells.

3. The cell system of Claim 2, wherein the umbilical mesenchymal cells are human umbilical mesenchymal cells.

4. The cell system of Claim 1, wherein the somatic cells are cells derived from ectoderm, mesoderm or endoderm.

5. The cell system of Claim 1, wherein the somatic cells are neurons.

6. The cell system of Claim 1, wherein the somatic cells are cardiomyocytes.

7. The cell system of Claim 1, wherein the somatic cells are cardiovascular cells.

8. A method of generating somatic cells comprising the steps:
preparing umbilical mesencymal cells from an umbilical cord;
culturing umbilical mesenchymal cells: and
inducing cell differentiation of the cultured umbilical mesenchymal cells.

9. The method of Claim 8, wherein the umbilical mesenchymal cells are mammalian umbilical mesenchymal cells.

10. The method of Claim 9, wherein the umbilical mesenchymal cells are human umbilical mesenchymal cells.

11. The method of Claim 8, wherein the somatic cells are cells derived from ectoderm, mesoderm or endoderm.

12. The method of Claim 8, wherein the umbilical mesenchymal cells are treated with *β*-mercaptoethanol (BME) to induce differentiation into neurons.

13. The method of Claim 8, wherein the umbilical mesenchymal cells are cultured in neuron-cultured 10%FCS-DMEM to induce differentiation into neurons.

14. The method of Claim 8, wherein the umbilical mesenchymal cells are treated with 5-azacytidine to induce differentiation into cardiomyocytes.

15. The method of Claim 8, wherein the umbilical mesenchymal cells are treated with 5-azacytidine to induce differentiation into cardiovascular cells.

16. The method of claim 8, wherein the step of preparing the umbilical mesenchymal cells includes isolating the umbilical mesenchymal cells from mesenchymal tissue in the umbilical cord.
